(19) Europäisches Patentamt
European Patent Office
Office européen des brevets

(11) **EP 1 296 594 B1**

(12) **FASCICULE DE BREVET EUROPEEN**

(45) Date de publication et mention de la délivrance du brevet:
**02.03.2011   Bulletin 2011/09**

(21) Numéro de dépôt: **01984090.9**

(22) Date de dépôt: **05.07.2001**

(51) Int Cl.:
*A61B 5/113* $^{(2006.01)}$

(86) Numéro de dépôt international:
**PCT/FR2001/002160**

(87) Numéro de publication internationale:
**WO 2002/002013 (10.01.2002 Gazette 2002/02)**

(54) **PROCEDE ET DISPOSITIF DE DETERMINATION DE PARAMETRES PHYSIOLOGIQUES CARDIO-RESPIRATOIRES**

VERFAHREN UND VORRICHTUNG ZUM BESTIMMEN VON PHYSIOLOGISCHEN HERZ-ATEMPARAMETERN

METHOD AND DEVICE FOR DETERMINING CARDIOPULMONARY PHYSIOLOGICAL PARAMETERS

(84) Etats contractants désignés:
**AT BE CH CY DE DK ES FI FR GB GR IE IT LI LU MC NL PT SE TR**

(30) Priorité: **05.07.2000   FR 0008743**

(43) Date de publication de la demande:
**02.04.2003   Bulletin 2003/14**

(73) Titulaire: **R.B.I.**
**38240 Meylan (FR)**

(72) Inventeurs:
• **BAGHAI, Ramine**
**F-38000 Grenoble (FR)**

• **EBERHARD, André**
**F-38330 Biviers (FR)**

(74) Mandataire: **de Beaumont, Michel**
**Cabinet Michel de Beaumont**
**1, rue Champollion**
**38000 Grenoble (FR)**

(56) Documents cités:
**FR-A- 2 286 370      FR-A- 2 664 487**
**FR-A- 2 787 008      GB-A- 1 596 298**
**US-A- 6 015 388**

**Description**

La présente invention concerne le domaine de la surveillance cardio-respiratoire d'une personne et, plus particulièrement, un dispositif de mesure non-invasif de la respiration et autres paramètres physiologiques. La respiration d'un patient est souvent mesurée pendant son sommeil pour détecter, par exemple, des apnées nocturnes. Parmi les paramètres physiologiques cardio-respiratoires le plus souvent souhaités par le corps médical, on retrouve le volume respiratoire, le débit respiratoire, la fréquence cardiaque ainsi que la fréquence respiratoire.

La présente invention concerne plus particulièrement l'utilisation d'un pléthysmographe à spires inductives. Un tel appareil est le plus souvent constitué d'une bande comprenant une spire thoracique et d'une bande comprenant une spire abdominal. Chacune des spires est à section variable et détermine la fréquence d'un oscillateur associé, solidaire de la bande. Les spires sont à section variable grâce à une disposition en sinusoïde dans la largeur des bandes. Les oscillateurs sont reliés à un circuit de traitement comprenant notamment un convertisseur fréquence-tensian par oscillateur. Les convertisseurs fournissent des tensions représentatives de la section du thorax et de la section de l'abdomen. La somme de ces deux tensions peut être rendue représentative du volume pulmonaire du patient pour surveiller sa respiration.

Les spires inductives thoracique et abdominale peuvent être intégrées dans un gilet dit respiratoire tel que décrit dans la demande de brevet français n° 2787008. Le gilet est fabriqué dans un tissu inextensible dans le sens de la hauteur. Pour éviter un retroussement du gilet qui provoquerait un déplacement des spires vers le haut, celui-ci est muni d'un entrejambe, par exemple, un morceau du tissu du gilet à fixer par pression ou bien une sangle que l'on peut tendre.

Un inconvénient des pléthysmographes à spires inductives classiques est qu'ils ne permettent pas d'obtenir le débit respiratoire du patient mais simplement une information représentative de son volume respiratoire. Pour retrouver l'information de débit, on doit revenir à des dispositifs encore antérieurs, tels qu'un spirographe ou un pneumotachographe permettant de mesurer les débits nasal et buccal. Un inconvénient de ce type de dispositif, généralement constitué d'un masque, est qu'il est trop contraignant à porter.

On connaît également des dispositifs de mesure de paramètres cardio-respiratoires pour obtenir non seulement une information liée au volume respiratoire par un pléthysmographe, mais également une information relative à la fréquence cardiaque. De tels dispositifs sont décrits, par exemple, dans les demandes internationales WO 86/00793 et WO 88/02237. Pour l'obtention des mesures autres que respiratoires, on y prévoit des capteurs supplémentaires dont les résultats de mesure sont, le cas échéant, exploités par une même unité centrale.

Un inconvénient de ces dispositifs est que la multiplication des capteurs nuit au confort du patient.

La demande de brevet N° 2 787 008 susmentionnée décrit un dispositif de mesure de respiration dans lequel un signal représentatif du volume pulmonaire d'un patient est obtenu en affectant des coefficients à des amplitudes respiratoires mesurées au moyen de spires inductives thoracique et abdominale d'un pléthysmographe.

Le brevet américain N° 6 015 388 décrit un procédé d'analyse de formes d'ondes respiratoires ayant pour but de déterminer des pics de débit inspiratoire et non une information continue relative au débit respiratoires du patient.

Un objet de la présente invention est de proposer un procédé et un dispositif de surveillance de données physiologiques cardio-respiratoires qui pallient les inconvénients des solutions connues.

La présente invention vise, plus particulièrement, à proposer un dispositif non-invasif et confortable permettant de fournir, de façon fiable, le débit respiratoire.

L'invention vise également à proposer un système autorisant une mesure en continu des paramètres physiologiques.

L'invention vise également à obtenir une surveillance des pulsations cardiaques sans capteur supplémentaire par rapport à un pléthysmographe à spires inductives.

La présente invention vise en outre à optimiser l'étalonnage d'un tel système de surveillance de données physiologiques.

Pour atteindre ces objets, la présente invention priévoit un procédé de surveillance de données physiologiques au moyen d'un pléthysmographe à spires inductives thoracique et abdominale, comportant les étapes de mesurer les amplitudes respiratoires d'un patient, sommer ces amplitudes respiratoires affectées d'un coefficient de pondération respectif pour obtenir un signal représentatif du volume respiré, effectuer in filtrage dudit signal ; et obtenir la fréquence cardiaque du patient du signal filtré.

Selon un mode de réalisation de la présente invention, le filtrage est de type passe-bande avec une bande passante approximativement comprise ente 2/3 de Hertz et 4 Hertz.

Selon un mode de réalisation de la présente invention, on effectue un filtrage passe-bas ayant une fréquence de coupure inférieure à 20 Hertz et, de préférence, de l'ordre de 10 Hertz, du signal représentatif du volume respiré avant ledit filtrage passe bande.

Selon un mode de réalisation de la présente invention, on effectue une dérivation du signal résultant dudit filtrage passe-bas et on applique un filtrage de type coupe-bande avec des fréquences de coupure d'approximativement

EP 1 296 594 B1

2/3 de Hertz et 12 Hertz au résultat de l'étape de dérivation précédente pour obtenir le débit respiratoire du patient.

L'invention prévoit également un appareil de surveillance de données physiologiques au moyen d'un pléthysmographe à spires inductives thoracique et abdominale comportant des moyens de mesure des amplitudes respiratoires d'un patient ; des moyens pour affecter un coefficient de pondération respectif aux amplitudes respiratoires mesurées ; des moyens pour sommer ces amplitudes respiratoires affectées de leur coefficient de pondération respectif, ces moyens fournissant un signal représentatif du volume respiré ; des moyens de traitement numérique dudit signal ; et des moyens pour obtenir la fréquence cardiaque du patient à partir de moyens de filtrage que comportent lesdits moyens de traitement numérique.

L'invention prévoit également un appareil dans lequel lesdits moyens de filtrage sont constitués d'un filtre de type passe-bande ayant une bande-passante (approximativement comprise entre 2/3 et 4 Hertz).

Selon un mode de réalisation de la présente invention, les spires inductives sont intégrées dans un gilet textile à mailles inextensibles dans le sens de la hauteur.

Selon un mode de réalisation de la présente invention, l'appareil comporte en outre un capteur de position du patient.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :

la figure 1 représente, de façon très schématique, un pléthysmographe à spires inductives du type auquel s'applique la présente invention ;
la figure 2 illustre, sous forme de schéma-bloc, un mode de mise en oeuvre du procédé de surveillance de données physiologiques selon la présente invention ;
la figure 3 représente un exemple de chronogrammes successivement obtenus par la mise en oeuvre du procédé de l'invention ;
la figure 4 représente un dispositif l'étalonnage qui n'est pas selon la présente invention ; et
la figure 5 représente, de façon très schématique, un mode de réalisation d'une interface de traitement de signaux d'un pléthysmographe selon la présente invention.

Les mêmes éléments ont été désignés par les mêmes références aux différentes figures. Pour des raisons de clarté, seuls les éléments d'un pléthysmographe qui sont nécessaires à la compréhension de l'invention ont été représentés aux figures et seront décrits par la suite.

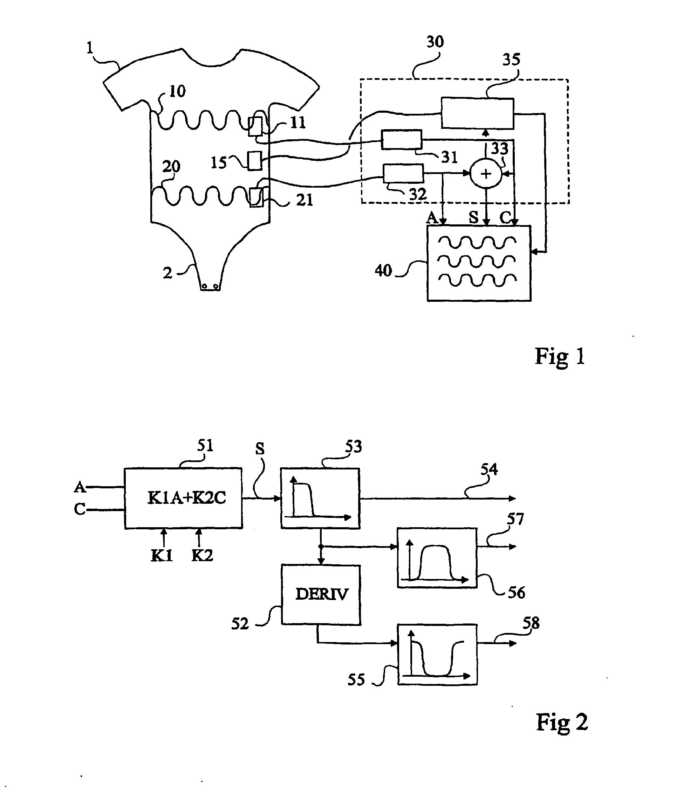

La figure 1 représente un mode de réalisation d'un pléthysmographe à spires inductives selon l'invention. De façon classique, comme cela est décrit par exemple dans la demande de brevet français n° 2787008, l'appareil est composé d'un gilet 1 intégrant une spire thoracique 10 et une spire abdominale 20. Chacune des spires est à section variable et détermine la fréquence d'un oscillateur associé, respectivement 11 ou 21. Les spires sont à section variable grâce à une disposition en sinusoïde dans la largeur du gilet. De préférence, le gilet 1 est fabriqué dans un tissu inextensible dans le sens de la hauteur. Le gilet est, comme indiqué précédemment, muni d'un entrejambe 2. Les oscillateurs 11 et 21 sont reliés à un circuit de traitement 30 comprenant un convertisseur fréquence-tension par oscillateur. Le convertisseur 31 fournit une tension C représentative de la section du thorax, et le convertisseur 32 fournit une tension A représentative de la section de l'abdomen. Les tensions C, A et leur somme pondérée S, fournie par un sommateur 33, peuvent être visualisées par tout dispositif 40 de mesure de tension tel qu'un voltmètre, un oscilloscope, un enregistreur graphique, etc.

La sommé S peut être rendue représentative du volume pulmonaire du patient en réglant convenablement des coefficients de pondération K1 et K2 des signaux A et C. En général, les coefficients K1 et K2 sont réglés lors d'une phase d'étalonnage. Par exemple, une première étape de la phase d'étalonnage sert à fixer ces coefficients, et une deuxième étape sert à déterminer une échelle pour que les valeurs de tension mesurées correspondent à des unités volumiques.

De préférence, la phase d'étalonnage comprend des étapes consistant à étalonner des spires pour chaque posture du patient (sur le dos, sur le ventre, sur le côté droit, sur le côté gauche) afin d'obtenir une mesure de la respiration utilisant un étalonnage correspondant à la position fournie par un capteur de position. Ainsi, le gilet est également associé à un capteur 15 (figure 1) de position du patient fournissant des informations à destination du circuit 30 de traitement pour qu'il adapte les résultats mesurés en fonction de la posture du patient. L'intégration d'un détecteur de position et exploitation dans un pléthysinographe à spires inductives sont décrites, par exemple, dans la demande de brevet N° 2 787 008 susmentionnée.

Une caractéristique de la présente invention est de prévoir, au sein du circuit 30 d'exploitation des signaux issus des oscillateurs 11 et 21, un traitement numérique particulier des signaux thoracique et abdominal. Ce traitement est illustré en figure 1 par un bloc 35 recevant la somme des signaux de tension thoracique C et abdominale A. En figure 1, le bloc 35 reçoit également le signal de position 15 pour adapter l'étalonnage du système et les mesures en fonction de la position du patient.

La figure 2 représente, sous forme de blocs fonctionnels, un mode de réalisation préféré de la présente invention. Cette figure illustre le traitement numérique de signaux opéré, par exemple par le bloc 35 de la figure 1, sur le signal S de sortie de l'additionneur 33.

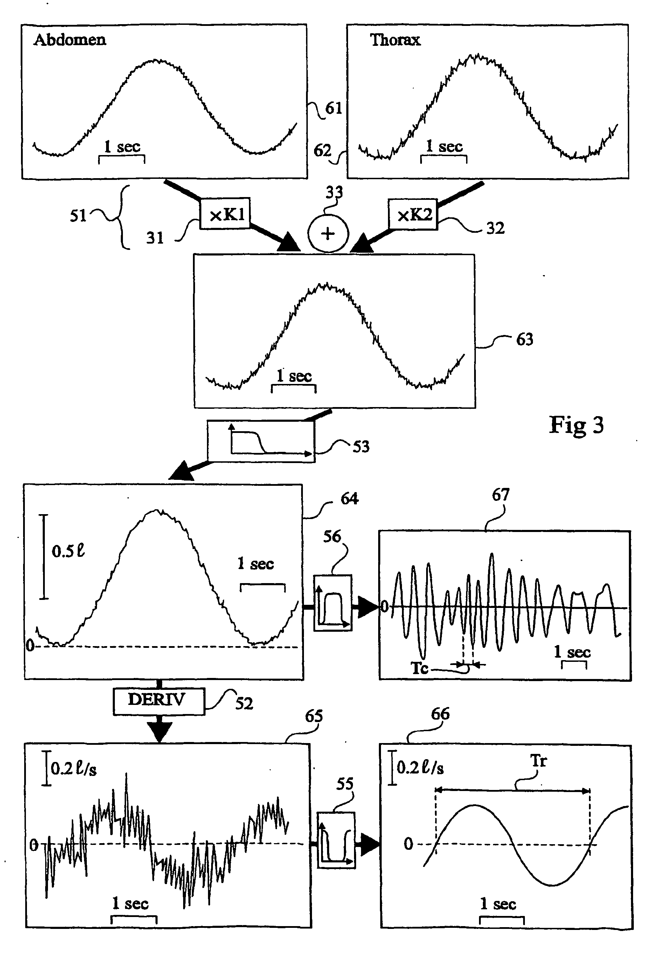

La figure 2 sera décrite avec la figure 3 qui illustre un exemple d'allures des différentes courbes de tension obtenues en fonction du temps en divers points du traitement numérique des signaux.

De façon classique, le procédé de traitement numérique du signal de l'invention commence par numériser, avec des gains respectifs K1 et K2, les tensions A et C des oscillateurs 11 et 21 (figure 1). Cette étape, suivie d'une sommation, est illustrée par un bloc 33 en figure 2. Les courbes 61 et 62 de la figure 3 illustrent un exemple d'allure de tensions mesurées A et C, respectivement à l'abdomen et au thorax au moyen d'un gilet tel qu'illustré par la figure 1. La courbe 63 représente le résultat de la sommation 33 de ces courbes. Cette courbe 63 est donc représentative du volume respiratoire du patient.

Selon la présente invention, pour obtenir un signal indicateur du débit respiratoire du patient, on dérive le signal de volume. Cette dérivation (bloc 52, DERIV) est, de préférence, précédée d'une étape de filtrage qui est choisie pour réaliser une fonction de filtre passe-bas. Cette étape est symbolisée par un bloc 53 qui délivre, sur une sortie 54, un signal 64 ayant sensiblement la même allure générale que la courbe 63 mais sur laquelle des perturbations haute fréquence ont été éliminées. Selon l'invention, ce signal 64 est délivré comme étant le volume respiratoire. La fréquence de coupure du filtre passe-bas est préférentiellement choisie pour être comprise entre 3 et 20 Hz, et est de préférence d'environ 10 Hz.

En dérivant le signal 64, on obtient un signal 65 fortement perturbé dans lequel tous les signaux parasites ont été amplifiés.

On aurait pu croire impossible d'extraire de cette courbe un signal exploitable représentatif du débit respiratoire. Or, les inventeurs ont pu constater que les perturbations amplifiées présentaient une répartition fréquentielle majoritairement supérieure au Hertz. Par conséquent, selon l'invention, le signal 65 est filtré (bloc 55) pour éliminer, en particulier, ces fréquences supérieures au Hertz et, de préférence, supérieures à 2/3 de Hertz. Selon un mode de réalisation préféré, on effectue, au lieu d'un filtrage passe-bas, un filtrage coupe-bande dans lequel on élimine les fréquences comprises entre approximativement 2/3 de Hertz et une dizaine de Hertz (de préférence entre 3/4 et 12 Hertz). En comparant les courbes obtenues par rapport à celles obtenues avec un appareil étalon, les inventeurs ont constaté qu'un filtre coupe-bande fournissait de meilleurs résultats qu'un filtre passe-bas. On obtient alors (sortie 58, figure 2) une courbe 66 représentative du débit respiratoire du patient. Cette courbe est, comme l'illustre la figure 3, parfaitement exploitable. Chaque valeur instantanée de la courbe 66 indique un débit instantané dont le signe indique s'il s'agit d'un débit inspiratoire ou expiratoire.

Comme il ressort de la figure 3, les courbes 64, 65 et 66 sont référencées par rapport à une origine. Cela indique qu'il s'agit de courbes étalonnées.

Une caractéristique d'un mode de réalisation préféré de la présente invention est d'utiliser le signal 64 représentant le volume respiratoire pour déterminer la pulsation cardiaque du patient. Pour ce faire, on réalise un filtrage passe-bande (bloc 56) dont la bande passante est approximativement comprise entre 2/3 de Hertz et 4 Hertz. Le résultat (sortie 57, figure 2) du filtrage passe-bande fournit une courbe 67 dont la fréquence du signal correspond à la fréquence des pulsations cardiaques du patient. Le filtrage passe-bande est représenté à la suite du filtre 53. Selon une variante non représentée, ce filtrage est effectué directement sur l'information de volume non filtrée (sortie du sommateur 33). Un avantage de cette variante est que l'on rend les dimensionnements des filtres d'obtention du débit et de la fréquence cardiaque indépendants les uns des autres. Selon une autre variante, s'appliquant au cas où le filtre d'extraction du rythme cardiaque est en aval du filtre passe-bas 53 qui présente alors une fréquence de coupure suffisamment basse, le rythme cardiaque est obtenu au moyen d'un filtre passe-haut dont la fréquence de coupure est d'environ 3/4 de Hertz.

En examinant les courbes de volume mesuré, que ce soit la courbe 63 avant filtrage ou la courbe 64 après filtrage, il paraissait impossible de pouvoir extraire du bruit considérable, un signal représentatif d'une donnée quelle qu'elle soit. Cette impression était corroborée par l'allure très fortement perturbée du signal dérivé 65. Contre toute attente, les inventeurs ont constaté que le signal présent dans la bande de fréquences habituelle des pulsations cardiaques était bien représentatif du rythme cardiaque du patient et était donc exploitable comme l'illustre la courbe 67. A partir de cette courbe, il suffit de mesurer la fréquence du signal, c'est-à-dire de détecter les changements de signe de ce signal, en tenant compte d'éventuelles perturbations subsistantes.

Par exemple, on pourra analyser le spectre fréquentiel du signal. On sait que la fréquence cardiaque est généralement comprise entre 3/4 de Hertz et 4 Hertz. On considère alors que la fréquence pour laquelle le spectre est maximum dans cette bande est la fréquence cardiaque moyenne sur la période d'analyse.

Une autre méthode consiste à détecter la période entre les maxima et minima successifs du signal 67 en tenant compte de seuils d'amplitude en deçà desquels on considère qu'il s'agit de bruit . La période médiane obtenue (en ne tenant pas compte des éventuelles extrêmes non significatives) donne la période des pulsations cardiaques.

Les deux méthodes ci-dessus sont, bien entendu, combinables.

Un avantage de la présente invention est qu'en mesurant seulement le volume respiratoire au moyen d'un pléthysmographe à spires inductives, on obtient au moins deux grandeurs physiologiques différentes. Plus précisément, on peut mesurer en continu, non seulement le volume respiratoire comme dans les systèmes classiques, mais également soit le débit respiratoire, soit la fréquence cardiaque, soit les deux. Pour simplifier l'exposé, un seul cycle respiratoire a été représenté en figure 3, mais la mesure s'effectue en continu.

Un autre avantage est qu'elle minimise le nombre de capteurs à imposer au patient.

La présente invention permet même d'isoler aisément une autre grandeur physiologique qui est la fréquence respiratoire du patient. Dans les systèmes classiques, cette fréquence respiratoire est isolée par un traitement du signal du volume respiratoire en détectant des changements de signe par rapport à une origine placée arbitrairement sur la courbe 63. Selon un mode de réalisation préféré de l'invention, on utilise le signal de débit 66 pour déterminer la fréquence respiratoire. En effet, cette détermination est alors beaucoup plus simple. Il suffit de détecter la fréquence du signal de débit qui, par l'opération de dérivée (52), se trouve automatiquement placée sur une origine (dérivée nulle) correspondant aux changements de pente du signal de volume. Ainsi, il est très facile d'isoler la période Tr du signal respiratoire et d'en déduire la fréquence.

Bien entendu, ce qui a été exposé ci-dessus en faisant référence par souci de clarté à des signaux analogiques correspond en pratique à un traitement numérique du signal, les tensions mesurées étant numérisées par les convertisseurs 31 et 32 en entrée du système. De plus, s'agissant de signaux numériques, ce qui a été illustré par un sommateur 33 et des blocs en figures 1 et 2 pourra, en pratique, être réalisé sous forme logicielle

Selon un mode de réalisation de l'invention, les filtres appliqués aux signaux traités réalisent un filtrage par convolution à portée limitée dans le domaine des temps. En d'autres termes, chaque échantillon k d'un signal est remplacé par une moyenne pondérée $\tilde{g}(k)$ de son voisinage, englobant tous les échantillons dont le numéro d'ordre est compris entre k-p et k+p. Cela revient à appliquer la formule suivante :

$$\tilde{g}(k) = \sum_{r=-p}^{+p} \omega_r \cdot g(k-r) ,$$

où $\omega_r$ représente le poids de l'échantillon courant, et où g représente la valeur de l'échantillon.

A chaque type de filtrage (passe-bas, coupe-bande ou passe-bande) correspond un jeu de poids $\omega_r$ et une étendue -p à +p des échantillons à prendre en compte. On notera que s'agissant d'échantillons numériques, cela décrit la réponse impulsionnelle du filtre. Sur le plan fréquentiel, la fonction de transfert induite par la convolution ci-dessus dépend bien entendu de la fréquence d'échantillonnage (pas entre deux échantillons successifs). De préférence, tous les filtres ont une fonction de transfert réelle afin de ne pas introduire de déphasage entre l'entrée et la sortie. Par conséquent, dans la réponse impulsionnelle, les poids sont symétriques ($\omega_{-r} = \omega_r$ pour tout r). La détermination des caractéristiques des filtres numériques respectant la formule ci-dessus est à la portée de l'homme du métier à partir des indications fonctionnelles fournies. On tiendra compte du délai nécessaire au calcul de la moyenne pondérée en fonction du nombre de coefficients pris pour le filtre (l'étendue -p à +p). Cela induit un retard dans la fourniture du résultat qui est fonction de la fréquence d'échantillonnage et du nombre d'échantillons futurs qui doivent être pris en compte pour le calcul de la moyenne pondérée affectée à l'échantillon courant. En pratique, un nombre d'échantillons comprise entre environ 20 et environ 60 de part et d'autre de l'échantillon courant donne des résultats tout à fait satisfaisants. Comme la fréquence d'échantillonnage est généralement de l'ordre de 30 à 50 Hz, le retard dépassera rarement une seconde, ce qui est parfaitement acceptable.

La mise en oeuvre de l'invention requiert d'étalonner le système avant d'effectuer une mesure, comme cela est le cas pour les systèmes classiques. Plusieurs méthodes d'étalonnage sont envisageables. Elles ont toutes en commun d'utiliser un dispositif étalon dont un masque est placé sur la bouche et le nez du patient pour obtenir des courbes étalons. On cherche alors à faire correspondre le mieux possible les courbes fournies par le pléthysmographe pendant les mêmes cycles respiratoires et ces courbes étalons.

Selon un mode de réalisation, une première étape de l'étalonnage consiste à choisir, par exemple sur un cycle, la meilleure combinaison des coefficients K1 et K2 pour que l'allure (sans respect d'échelle) de la somme des signaux corresponde à l'allure d'un volume réel. Cela revient à fixer le rapport K1/K2. Le volume réel est classiquement intégré à partir d'une mesure de débit. Il est connu que le choix d'un rapport K1/K2 sensiblement égal à 2 fournit souvent de bons résultats. Par conséquent, dans un tel mode de réalisation simplifié, un seul coefficient doit être déterminé lors de la phase d'étalonnage. Selon un autre mode de réalisation, les coefficients K1 et K2 seront calculés en appliquant une méthode dite des "moindres carrés" comme on le verra par la suite.

Le principe de l'étalonnage des unités d'un volume ou d'un débit consiste à appliquer la règle de transformation suivante :

U = α (N - Z), où U représente la valeur du signal étalonné exprimé dans un système d'unité dépendante de sa nature (dans le cas d'un débit, celui-ci peut être exprimé en litre par seconde, par exemple), où N représente la grandeur numérique fournie par le convertisseur analogique-numérique du signal, où Z représente la grandeur numérique fournie par le convertisseur quand le signal est nul, et où α représente le facteur d'échelle. L'étalonnage en unité de valeur consiste à déterminer les deux grandeurs α et Z. La valeur Z s'obtient généralement en effectuant une moyenne des valeurs fournies par le convertisseur analogique/numérique sur une plage où le signal est nul.

L'obtention du facteur d'échelle α nécessite de disposer d'une grandeur étalon.

Selon une première méthode d'étalonnage de l'invention, on utilise un pneumotachographe qui constitue un appareil parfaitement connu de mesure du débit respiratoire. Une caractéristique de cette première méthode est que les coefficients de pondération ne sont pas fixés arbitrairement mais optimisés par l'étalonnage.

Pour le pneumotachographe, le facteur d'échelle a peut être obtenu de plusieurs manières.

Selon un premier exemple connu, on établit un débit d'air constant dans un dispositif de type diaphragme offrant une résistance à la pression. Cette résistance provoque, entre l'entrée et la sortie du diaphragme, une chute de pression. On mesure cette chute de pression grâce à la différence des niveaux d'eau contenus dans un tube en forme de U. Le diaphragme ayant été préalablement calibré par une autre méthode, on connaît la loi qui, à la différence de niveau d'eau, fait correspondre le débit exprimé en unité habituelle (par exemple des litres par seconde).

Selon un deuxième exemple connu, on utilise une seringue de volume connu et on convertit la courbe du débit pendant que l'on vide cette seringue.

Une fois étalonné, le pneumotachographe est utilisé pour mesurer le débit respiratoire du patient en parallèle avec la mesure du volume au moyen du pléthysmographe. On effectue alors une détermination des coefficients K1 et K2 en appliquant une méthode dite des "moindres carrés". Cela revient à choisir les coefficients K1 et K2 tels que la somme des carrés des écarts entre le volume reconstitué par le pléthysmographe (K1A + K2C) et le volume fourni par l'intégration des résultats du pneumotachographe, soit minimale. En d'autres termes, on cherche, par approximations successives, à rendre l'expression suivante minimale :

$$\sum_{i=1}^{n} \left( K1 \cdot A_i + K2 \cdot C_i - \dot{V}_i \right)^2 ,$$

où V représente le volume entrait du débit fourni par le pneumotachographe, et où i désigne le numéro d'ordre de l'échantillon dans une série de n échantillons pris pour étalonner l'appareil.

La mise en pratique d'une telle méthode d'étalonnage est à la portée de l'homme du métier à l'aide des moyens de traitement numériques connus (par exemple, un micro-ordinateur convenablement programmé).

En variante, le rapport entre les coefficients K1 et K2 sera fixé (par exemple K = K1/K2 = 2 ) et c'est le coefficient K qui sera détermine par la méthode des moindres carrés en minimisant la somme suivante :

$$\sum_{i=1}^{n} \left( K \cdot (A_i + 2 \cdot C_i) - V_i \right)^2 .$$

Selon une deuxième méthode d'étalonnage de l'invention, on cherche à étalonner le pléthysmographe directement à partir d'une mesure de volume. On doit alors utiliser un dispositif d'étalonnage du volume respiratoire par une comparaison directe (sans intégration) par rapport à un volume étalon. Cette deuxième méthode requiert, dans une première étape, de fixer arbitrairement le rapport entre les coefficients de pondération (par exemple, K1 = 2K2).

Dans une deuxième étape, on peut utiliser une seringue classique de volume connu que l'on relie au masque du patient. A chaque cycle respiratoire, la seringue étalon force un volume d'inspiration du patient qui, pour permettre l'étalonnage, doit inspirer l'intégralité du volume de la seringue étalon. L'amplitude du signal inspiratoire obtenue par le pléthysmographe correspond alors au volume étalon.

L'utilisation d'une seringue ne constitue pas un mode de réalisation préféré de cette deuxième méthode. En effet, les inventeurs considèrent que les résultats d'étalonnage donnés par cette méthode classique sont très approximatifs. De plus, l'utilisation de la seringue étalon impose au patient de forcer ses inspirations et expirations. L'effort fourni par le patient modifie sa respiration par rapport à sa respiration naturelle, ce qui constitue une source d'erreur.

Par conséquent, selon un mode de mise en oeuvre préféré de la deuxième méthode d'étalonnage, l'invention prévoit d'utiliser un dispositif d'étalonnage particulier du volume respiratoire qui pallie ces difficultés.

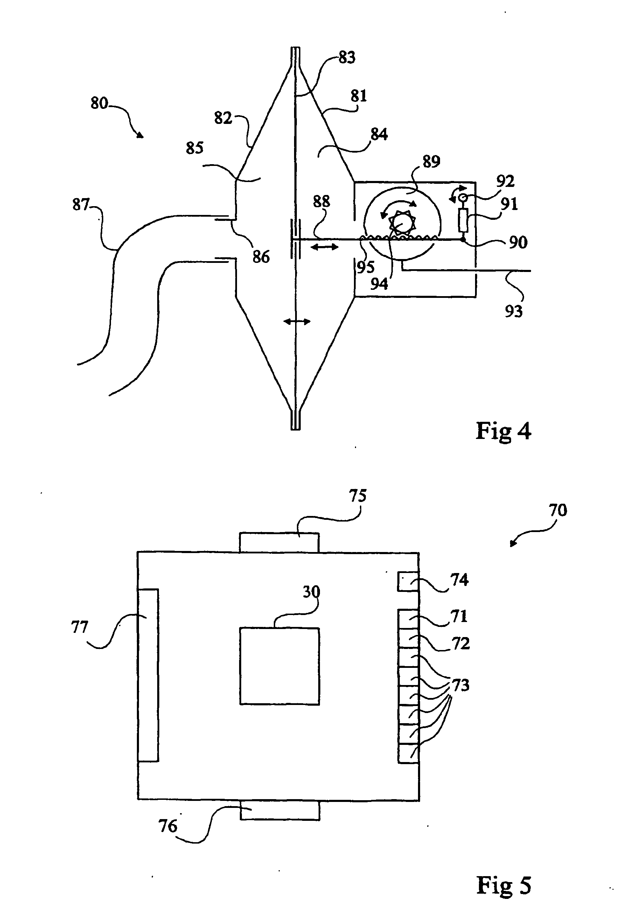

La figure 4 représente, par une vue schématique en coupe, un dispositif étalon qui n'est pas selon l'invention.

Ce dispositif 80 comporte une enceinte étanche à l'air, par exemple, constituée de deux parois 81 et 82 en forme de soucoupes réunies par leurs périphéries respectives. Le volume interne de l'enceinte 80 est divisé par une membrane déformable et imperméable 83, qui définit deux chambres 84 et 85. Une chambre 84, dite de mesure, comporte un orifice 86 de liaison étanche à un tube 87 de mesure. Le tube 87 est, par exemple, destiné à être relié à un masque (non représenté) appliqué sur la face du patient. L'autre chambre 85, dite chambre étalon, comporte un capteur des déplacements de la membrane. Ce capteur est à frottement minimum pour minimiser les risques de perturbation des mesures. Par exemple, il s'agit d'une tige 88 reliée par une première extrémité au centre de la membrane 83, et coopérant avec un codeur incrémentiel 89 ou un potentiomètre. La deuxième extrémité de la tige 88 peut être reliée, par une articulation 90, à une première extrémité d'un ressort 91 dont l'autre extrémité est fixée, par une articulation 92, à une paroi de la chambre 85. Ce mode de réalisation suppose que l'élasticité de la membrane n'est pas suffisante pour retrouver la position de repos. Dans le cas contraire, aucun dispositif de rappel n'est nécessaire.

De préférence, le codeur 89 est contenu dans le volume de la chambre étalon 85. Cela évite à la tige 88 de traverser un joint d'étanchéité qui introduirait des frottements néfastes aux mesures. Ainsi, la chambre 85 comporte, de préférence, un prolongement vers l'arrière de la tige (sa deuxième extrémité). Seule une liaison électrique 93 fournissant les résultats de déplacement convertis par le codeur 89 traverse la paroi de la chambre 85. Le fonctionnement et la réalisation d'un codeur incrémentiel associé à une tige de mesure de déplacement sont parfaitement connus. Par exemple, le codeur 89 comporte une roue dentée 94 pour coopérer avec des dents 95 que comporte la tige 88 dans une portion de sa longueur.

Les déplacements respectifs possibles des différents constituants du dispositif étalon de l'invention ont été illustrés par des flèches en figure 4. Les déplacements de la tige 88 sont canalisés, par sa liaison au codeur 89 et au ressort 91, dans une direction perpendiculaire au plan de la membrane 83.

Quand le patient respire, cela provoque un déplacement (dans un sens ou dans l'autre selon qu'il inspire ou expire) de la membrane 83. Ce déplacement est converti en signal électrique (de préférence numérique) par le codeur 89. Compte tenu de la forme circulaire de la membrane 83, son déplacement latéral h correspond à une variation de volume $V = \pi R^2 h/3$, où R représente le rayon de la membrane. A partir de la position de repos de la membrane, on peut donc déterminer très précisément et directement les volutes respirés .

Un avantage du dispositif étalon de l'invention est que le patient peut respirer de façon naturelle. L'étalonnage s'effectuant sur un faible nombre de cycles de respiration, il n'est pas gênant que le patient ne renouvelle pas son air pendant l'étalonnage.

L'utilisation du dispositif étalon de l'invention améliore considérablement la fiabilité d'un pléthysmographe à spires inductives. En outre, l'étalonnage est particulièrement simple et confortable pour le patient (en particulier, pour un patient ayant des difficultés respiratoires ou auquel on ne peut demander de forcer sa respiration, par exemple, parce qu'il est inconscient) . On évite également le recours aux équipements complexes et coûteux du type pneumotachographe.

Selon un mode de réalisation préféré de l'invention, on utilise un circuit d'interface dédié (le circuit 30, figure 1) entre le gilet 1 à spires inductives et un afficheur ou mémorisateur (40, figure 1) .

La figure 5 représente, de façon très schématique et par ses entrées-sorties, un mode de réalisation d'un boîtier d'interface 70 selon la présente invention.

Ce boîtier 70 comprend un circuit 30 de traitement du signal basé sur un microprocesseur (par exemple, un microprocesseur 8 bits) . Les entrées de signaux comprennent essentiellement une borne 71 d'entrée du signal issu de l'oscillateur 11, une borne 72 d'entrée du signal issu de l'oscillateur 21 et des bornes d'entrées 73 associées à l'élément de positionnement 15. S'agissant d'un système à bille tel que décrit dans la demande de brevet français n˚ 2787008, on utilise cinq bornes d'entrées plus une borne de référence. De préférence, on prévoit également une borne d'entrée 74 destinée à recevoir un signal de débit étalonné provenant, par exemple, d'un pneumotachographe. Ainsi, le circuit d'interface 70 de l'invention sert à organiser les échanges entre les différents appareils, que ce soit pendant les phases de mesure ou pendant les phases d'étalonnage. Le circuit comprend une liaison série (symbolisée par son accès 75) vers un appareil d'exploitation des mesures (non représenté), par exemple, un micro-ordinateur. Le boîtier 70 nécessite d'être alimenté et comprend donc un ou plusieurs contacts 76 vers un bloc d'alimentation (non représenté) .

Ainsi décrit, le circuit d'interface 70 de l'invention comprend tous les éléments nécessaires. Toutefois, il peut être souhaitable de disposer des grandeurs analogiques mesurées afin de les visualiser sur des afficheurs classiques prévus pour recevoir ce type de données. Par conséquent, dans un mode de réalisation préféré, on prévoit une conversion numérique-analogique des mesures et déterminations effectuées. Ces différentes mesures sont restituées sur un bornier 77 délivrant des signaux analogiques. Il s'agira, par exemple, des allures analogiques des tensions de la spire inductive du thorax, de la spire inductive de l'abdomen, du signal de volume (54, figure 2), du signal de position, du débit (58, figure 2), de la fréquence cardiaque (signal 57, figure 2), de la fréquence respiratoire, etc.

Bien entendu, la présente invention est susceptible de diverses variantes et modifications qui apparaîtront à l'homme de l'art. En particulier, la réalisation pratique des circuits nécessaires à la mise en oeuvre du procédé de l'invention est à la portée de l'homme du métier à partir des indications fonctionnelles données ci-dessus. De même, le regroupement des différentes fonctions au sein d'un même boîtier d'interface se déduit de ce qui précède et des techniques connues. Par ailleurs, la réalisation pratique des filtres numériques utilisés est à la portée de l'homme du métier à partir des indications fonctionnelles données dans la présente description. De plus, bien que l'on ait fait référence à des méthodes d'étalonnage préférées, on pourra mettre en oeuvre d'autres méthodes classiques pour étalonner un système de l'invention. En outre, si dans une application préférée, le pléthysmographe à spires inductives est intégré dans un gilet porté par le patient, le procédé de traitement de signaux de l'invention peut plus généralement être mis en oeuvre avec un pléthysmographe à spires inductives ayant des bandes séparées.

**Revendications**

1. Procédé de surveillance de données physiologiques au moyen d'un pléthysmographe à spires inductives thoracique (10) et abdominale (20), comportant les étapes de :

   mesurer (31, 32) les amplitudes respiratoires (61, 62) d'un patient ;
   sommer (33) ces amplitudes respiratoires affectées d'un coefficient de pondération respectif (K1, K2) pour obtenir un signal (63) représentatif du volume respiré ;
   effectuer un filtrage (56) dudit signal ; et
   obtenir la fréquence cardiaque (67) du patient du signal filtré.

2. Procédé selon la revendication 1, **caractérisé en ce que** le filtrage est de type passe-bande avec une bande passante comprise entre 2/3 de Hertz et 4 Hertz.

3. Procédé selon la revendication 1 ou 2, dans lequel un filtrage passe-bas (53) ayant une fréquence de coupure inférieure à 20 Hertz et, de préférence, de l'ordre de 10 Hertz, dudit signal représentatif du volume respiré (63) est effectué avant ledit filtrage passe-bande.

4. Procédé selon la revendication 3, comportant en outre les étapes :

   d'effectuer une dérivation (52) du signal résultant dudit filtrage passe-bas ; et
   appliquer un filtrage (55) de type coupe-bande avec des fréquences de coupure entre 2/3 de Hertz et 12 Hertz au résultat de l'étape de dérivation précédente pour obtenir le débit respiratoire (66) du patient.

5. Appareil de surveillance de données physiologiques au moyen d'un pléthysmographe à spires inductives thoracique (10) et abdominale (20), comportant :

   des moyens de mesure (31, 32) des amplitudes respiratoires (61, 62) d'un patient ;
   des moyens (31, 32) pour affecter un coefficient de pondération respectif (K1, K2) aux amplitudes respiratoires mesurées ;
   des moyens (33) pour sommer ces amplitudes respiratoires affectées de leur coefficient de pondération respectif, ces moyens fournissant un signal (63) représentatif du volume respiré ;
   des moyens de traitement numérique dudit signal; et
   des moyens pour obtenir la fréquence cardiaque du patient à partir de moyens de filtrage que comportent lesdits moyens de traitement numérique.

6. Appareil selon la revendication 5, dans lequel lesdits moyens de filtrage sont constitués d'un filtre de type passe-bande ayant une bande-passante comprise entre 2/3 et 4 Hertz.

7. Appareil selon la revendication 10, **caractérisé en ce que** les spires inductives sont intégrées dans un gilet textile (1) à mailles inextensibles dans le sens de la hauteur.

8. Appareil selon la revendication 10 ou 11, **caractérisé en ce qu'**il comporte en outre un capteur (15) de position du patient.

**Claims**

1. A method for monitoring physiological data by means of a plethysmograph with thoracic (10) and abdominal (20) inductive coils, comprising the steps of:

measuring (31, 32) a patient's respiratory amplitudes (61, 62);
summing up (33) these respiratory amplitudes assigned with a respective weighting coefficient (K1, K2) to obtain a signal (63) representative of the breathed volume;
performing a filtering (56) of said signal; and
obtaining the patient's heart rate (67) from the filtered signal.

2. The method of claim 1, **characterized in that** the filtering is of band-pass type with a passband comprised between 2/3 hertz and 4 hertz.

3. The method of any of claims 1 or 2, wherein a low-pass filtering (53) having a cut-off frequency smaller than 20 hertz and, preferably, on the order of 10 hertz, of said signal (63) representative of the breathed volume is carried out before said band-pass filtering.

4. The method of claim 3, further comprising the steps of:

deriving (52) the signal resulting from said low-pass filtering; and
applying a filtering (55) of a band-stop type with cut-off frequencies of approximately 2/3 hertz and 12 hertz to the result of the former filtering step to obtain the patient's respiratory flow (66).

5. A device for monitoring physiological data by means of a plethysmograph with thoracic (10) and abdominal (20) inductive coils, comprising:

means (31, 32) for measuring a patient's respiratory amplitudes (61, 62);
means (31, 32) for assigning a respective weighting coefficient (K1, K2) to the measured respiratory amplitudes;
means (33) for summing up these respiratory amplitudes assigned with their respective weighting coefficient, these means providing a signal (63) representative of the breathed volume;
means for a digital processing of said signal; and
means for obtaining the patient's heart rate from filtering means included in said digital processing means.

6. The device of claim 5, wherein the filtering means are of band-pass type with a passband comprised between 2/3 hertz and 4 hertz.

7. The device of claim 5, **characterized in that** the inductive coils are integrated in a textile jacket (1) with stitches which are unstretchable heightwise.

8. The device of claim 5 or 6, **characterized in that** it further comprises a sensor (15) of the patient's position.

**Patentansprüche**

1. Verfahren zum Überwachen physiologischer Daten mittels eines Plethysmographen mit thorakalen (10) und abdominellen (20) Induktionsspulen, welches folgende Schritte aufweist:

Messen (31, 32) der Atmungsamplituden (61, 62) eines Patienten;
Aufsummieren (33) dieser Atmungsamplituden, denen ein entsprechender Gewichtungskoeffizient (K1, K2) zugeordnet ist, um ein Signal (63) zu erhalten, welches für das Atmungsvolumen repräsentativ ist;
Durchführen einer Filterung (56) des Signals; und
Erhalten der Herzfrequenz (67) des Patienten aus dem gefilterten Signal.

2. Verfahren nach Anspruch 1, **dadurch gekennzeichnet, dass** es sich um eine Filterung vom Bandpass-Typ mit einem Durchlassbereich zwischen 2/3 Hertz und 4 Hertz handelt.

3. Verfahren nach einem der Ansprüche 1 oder 2, wobei eine Tiefpassfilterung (53) mit einer Grenzfrequenz kleiner als 20 Hertz, und vorzugsweise in der Größenordnung von 10 Hertz, des Signals (63), welches für das Atmungsvolumen repräsentativ ist, vor der Bandpassfilterung durchgeführt wird.

4. Verfahren nach Anspruch 3, welches ferner folgende Schritte aufweist:

Ableiten (52) des Signals, welches aus der Tiefpassfilterung resultiert; und

Anwenden einer Filterung (55) vom Bandsperrtyp mit Grenzfrequenzen von ungefähr 2/3 Hertz und 12 Hertz auf das Resultat des vorherigen Filterschrittes, um den Atemfluss (66) des Patienten zu erhalten.

5. Vorrichtung zum Überwachen physiologischer Daten mittels eines Plethysmographen mit thorakalen (10) und abdominellen (20) Induktionsspulen, welche Folgendes aufweist:

Mittel (31, 32) zum Messen der Atemamplituden (61, 62) eines Patienten;

Mittel (31, 32) zum Zuweisen eines entsprechenden Gewichtungskoeffizienten (K1, K2) an die gemessenen Atmungsamplituden;

Mittel (33) zum Aufsummieren dieser Atmungsamplituden, denen ihr entsprechender Gewichtungskoeffizient zugewiesen ist, wobei diese Mittel ein Signal (63) liefern, welches für das Atmungsvolumen repräsentativ ist;

Mittel zu einer digitalen Verarbeitung des Signals; und

Mittel zum Erhalten der Herzfrequenz des Patienten aus Filtermitteln, die in den digitalen Verarbeitungsmitteln enthalten sind.

6. Vorrichtung nach Anspruch 5, wobei es sich um Filtermittel vom Band-pass-Typ mit einem Durchlassbereich zwischen 2/3 Hertz und 4 Hertz handelt.

7. Vorrichtung nach Anspruch 5, **dadurch gekennzeichnet, dass** die Induktionsspulen in einer Textiljacke (1) mit Maschen integriert sind, die in der Höhe nicht dehnbaren sind.

8. Vorrichtung nach Anspruch 5 oder 6, **dadurch gekennzeichnet, dass** sie ferner einen Sensor (15) für die Position des Patienten aufweist.

Fig 1

Fig 2

Abdomen

Thorax

1 sec

1 sec

61

62

51

31

×K1

33

+

×K2

32

1 sec

63

53

64

67

0.5 ℓ

1 sec

0

56

0

Tc

1 sec

Fig 3

DERIV

52

65

66

0.2 ℓ/s

0

1 sec

55

0.2 ℓ/s

Tr

0

1 sec

**Fig 4**

**Fig 5**

**RÉFÉRENCES CITÉES DANS LA DESCRIPTION**

**Documents brevets cités dans la description**

- FR 2787008
- WO 8600793 A
- WO 8802237 A
- WO 2787008 A